# EUROPEAN PATENT APPLICATION

(11) **EP 4 723 284 A1**
(43) Date of publication of application: **08.04.2026**
(21) Application number: 23952063.8
(22) Date of filing: 30.11.2023
(51) Int. Cl.: H01M 10/0569, H01M 10/0525

(54) **ELECTROLYTE, SECONDARY BATTERY, AND ELECTRIC DEVICE**

(30) Priority: 15.09.2023 CN 202311191041
(71) Applicant: Contemporary Amperex Technology Co., Limited, Ningde, Fujian 352100 (CN)
(72) Inventor: FENG, Fuxiang, Ningde, Fujian 352100 (CN); WANG, Hansen, Ningde, Fujian 352100 (CN); WANG, Yuchun, Ningde, Fujian 352100 (CN); ZHANG, Rupeng, Ningde, Fujian 352100 (CN); WAN, Pan, Ningde, Fujian 352100 (CN)
(74) Representative: Gong, Jinping
(86) International application number: PCT/CN2023/135409
(87) International publication number: WO 2025/055140

(57) **Abstract**

An electrolyte, a secondary battery, and an electric device. The electrolyte comprises a component, a nitrile solvent. The nitrile solvent comprises a compound represented by formula (1): wherein: L is selected from any one of C(R₁R₂), O, Si(R₃R₄), B(R₅), P(R₆R₇R₈), and N(R₉); R₁-R₉ are each independently selected from at least one of H, halogen, substituted or unsubstituted alkyl having 1-3 carbon atoms, alkoxy having 1-3 carbon atoms, amino, and cyano, and R₁ and R₂ are not cyanos and H; R₁₁ is an alkane chain containing 1-4 carbon atoms; R₁₂-R₁₄ are each independently selected from at least one of H, halogen, substituted or unsubstituted alkyl having 1-3 carbon atoms, alkoxy having 1-3 carbon atoms, amino, and cyano; and when L is C(R₁R₂), R₁₂-R₁₄ are not cyanos.

## Description

### CROSS-REFERENCE

The present application refers to Chinese Patent Application No. 202311191041.9 entitled "ELECTROLYTIC SOLUTION, SECONDARY BATTERY, AND ELECTRIC DEVICE" and filed on September 15, 2023, which is incorporated herein by reference in its entirety.

### TECHNICAL FIELD

The present application relates to the technical field of batteries, and in particular, to an electrolytic solution, a secondary battery, and an electric device.

### BACKGROUND

Lithium batteries and other secondary batteries have been widely used as they are clean and renewable. Lithium-ion secondary batteries have been widely applied in various fields, such as consumer electronics, electric vehicles, and energy storage.

With the rapid development of the new energy industry, the demand for electric vehicles, electric bicycles, and other new energy transportation tools has been growing significantly, along with increasingly higher performance requirements. However, the energy density of conventional secondary batteries is approaching their theoretical limits. To meet future application scenarios requiring higher energy density, such as long-range electric vehicles and electric aircraft, lithium-metal secondary batteries with superior energy density have been developed. However, the lithium metal negative electrode in lithium-metal secondary batteries exhibits high reactivity and readily reacts with the electrolyte. This leads to the formation of a solid electrolyte interphase (SEI) layer with reduced mechanical strength and chemical stability, thereby triggering lithium dendrite growth. As a result, it causes lithium source consumption, shortens battery lifespan, and poses safety risks.

Therefore, the conventional techniques are to be further improved.

### SUMMARY

According to various embodiments of the present application, the present application provides an electrolytic solution, a secondary battery, and an electric device, aiming to improve the cycle performance of batteries.

The present application is realized by the following technical solutions.

In a first aspect of the present application, provided is an electrolytic solution. Components of the electrolytic solution include a nitrile solvent, and the nitrile solvent includes a compound represented by formula (1): 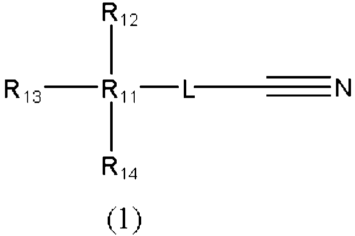
where L is selected from any one of C(R₁R₂), O, Si(R₃R₄), B(R₅), P(R₆R₇R₈), and N(R₉); R₁-R₉ are each independently selected from any one of H, halogen, substituted or unsubstituted alkyl with 1-3 carbon atoms, alkoxy with 1-3 carbon atoms, amine group, and cyano, and R₁ and R₂ are not cyano;
R₁₁ is an alkane chain containing 1-4 carbon atoms;
R₁₂-R₁₄ are each independently selected from any one of H, halogen, substituted or unsubstituted alkyl with 1-3 carbon atoms, alkoxy with 1-3 carbon atoms, amine group, and cyano; when L is selected from C(R₁R₂), R₁₂-R₁₄ are not cyano.

The electrolytic solution described above contains a nitrile solvent with a specific structure, and the compound represented by formula (1) contains an alkyl chain. When L is selected from O, Si(R₃R₄), B(R₅), P(R₆R₇R₈), and N(R₉), the alkyl carbon chain is separated from cyano by the groups, such that the activity of the group on the alkyl chain is reduced, and the redox stability of the nitrile solvent is improved; when L is selected from C(R₁R₂), that is, when cyano is directly linked to the alkyl chain, the groups of R₁ and R₂ are regulated, so that R₁ and R₂ are not cyano and H, and R₁₂-R₁₄ are not cyano. Thus, the reactivity of the carbon atom directly linked to cyano is reduced, and good redox stability is kept. As such, the compound represented by formula (1) with the specific structure described above not only exhibits good solubility for electrolyte salts but also maintains good oxidation stability. When applied to the preparation of secondary batteries, particularly lithium metal secondary batteries, it can reduce the likelihood of corrosion on the positive electrode metal, thereby enhancing the cycle performance of the battery.

In some of the embodiments, the nitrile solvent includes a compound represented by formula (1-1):

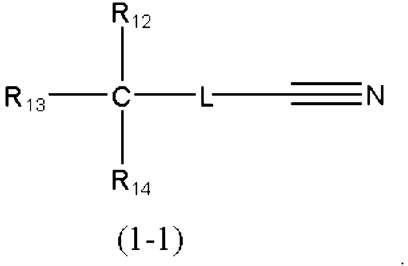

In some of the embodiments, L is selected from any one of C(R₁R₂), Si(R₃R₄), and N(R₉).

When L is selected from C(R₁R₂), Si(R₃R₄), and N(R₉), the compound represented by formula (1) can simultaneously achieve a wider melting point range, higher redox stability, and excellent lithium salt dissolving capability.

In some of the embodiments, the compound represented by formula (1) satisfies at least one of the following conditions (1)-(3):
(1) R₁-R₉ are each independently selected from any one of halogen, alkyl with 1-3 carbon atoms, halogen-substituted alkyl with 1-3 carbon atoms, alkoxy with 1-3 carbon atoms, and cyano;
(2) R₁ and R₂ are not H at the same time, R₃ and R₄ are not H at the same time, R₆, R₇, and R₈ are not H at the same time, and R₅ and R₉ are not H; and
(3) R₁₂-R₁₄ are each independently selected from any one of H, halogen, alkyl with 1-3 carbon atoms, halogen-substituted alkyl with 1-3 carbon atoms, alkoxy with 1-3 carbon atoms, and cyano.

In some of the embodiments, the nitrile solvent includes at least one of compounds represented by formula (1a) to formula (1f): 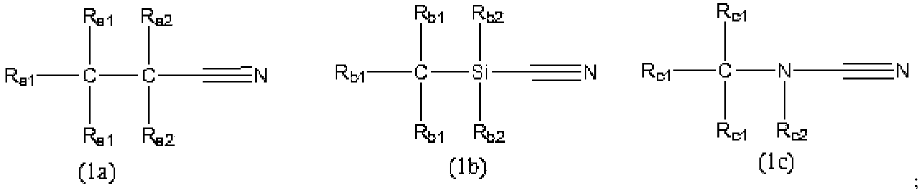 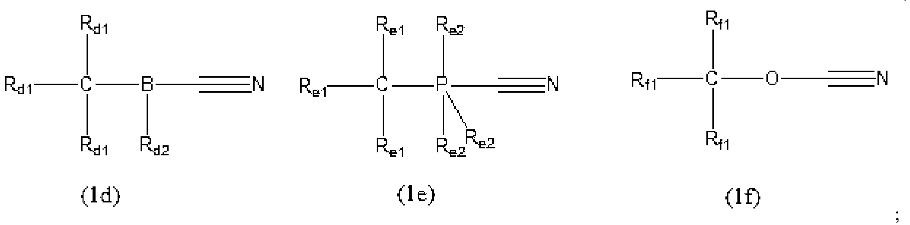
where each Rₐ₁, each R_{b1}, each R_{c1}, each R_{d1}, each Rₑ₁, and each R_{f1} are each independently selected from any one of H, halogen, alkyl with 1-3 carbon atoms, halogen-substituted alkyl with 1-3 carbon atoms, alkoxy with 1-3 carbon atoms, and cyano, and each Rₐ₁ is not cyano;
each Rₐ₂, each R_{b2}, each R_{c2}, each R_{d2}, and each Rₑ₂ are each independently selected from any one of halogen, alkyl with 1-3 carbon atoms, halogen-substituted alkyl with 1-3 carbon atoms, and alkoxy with 1-3 carbon atoms.

In some of the embodiments, the nitrile solvent satisfies at least one of the following conditions (1)-(6):
(1) at least one Rₐ₂ is alkoxy with 1-3 carbon atoms;
(2) each R_{b2} is independently selected from any one of alkyl with 1-3 carbon atoms and alkoxy with 1-3 carbon atoms;
(3) each R_{c1} is independently selected from alkyl with 1-3 carbon atoms;
(4) each R_{d1} is independently selected from alkyl with 1-3 carbon atoms;
(5) each Rₑ₁ is independently selected from alkoxy with 1-3 carbon atoms; and
(6) each R_{f1} is independently selected from any one of H and alkyl with 1-2 carbon atoms.

In some of the embodiments, the nitrile solvent includes at least one of compounds represented by formula (11) to formula (58):

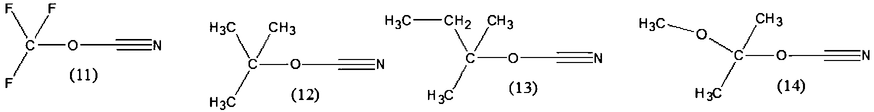

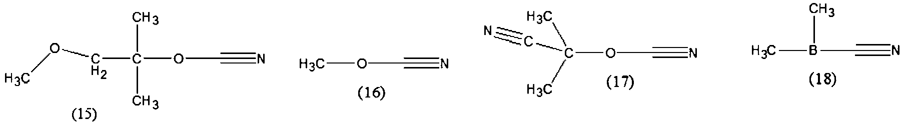

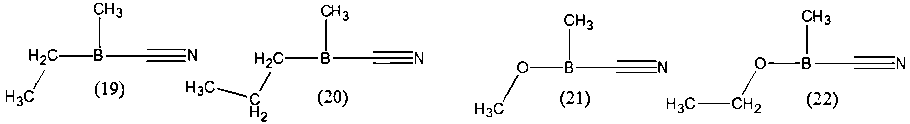

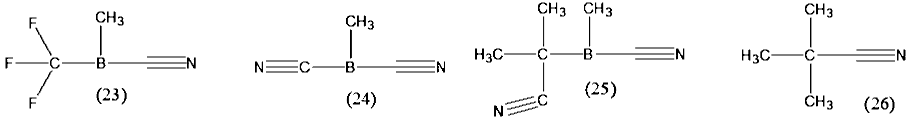

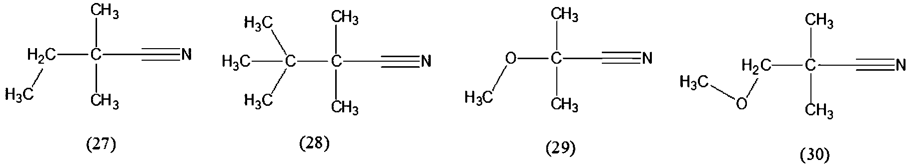

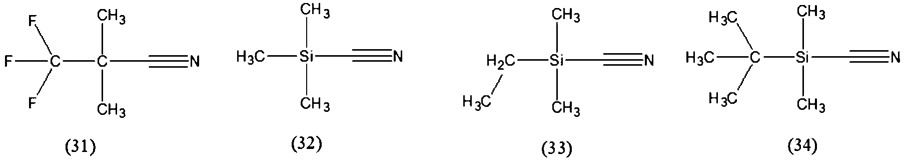

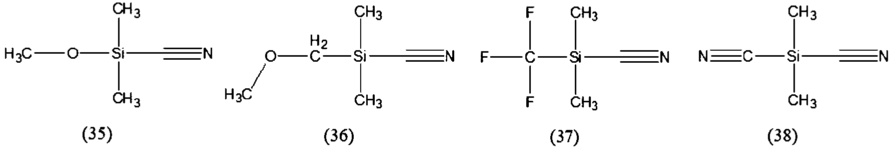

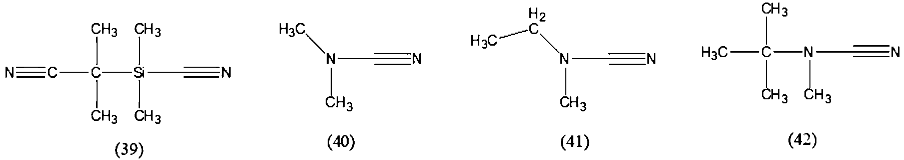

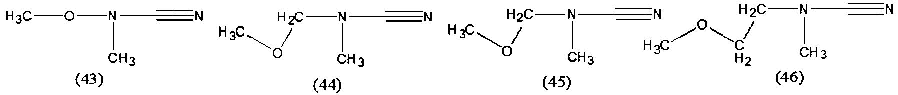

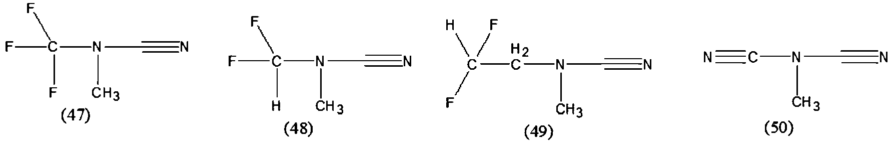

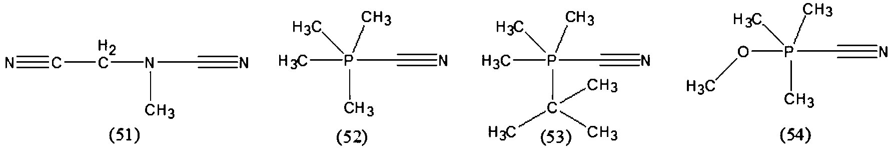

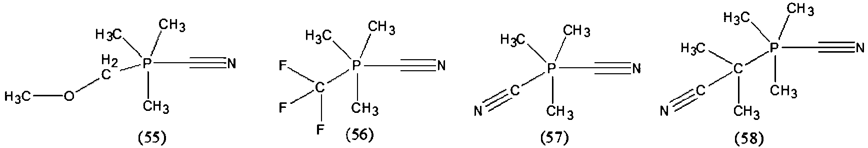

In some of the embodiments, the components of the electrolytic solution further include an electrolyte salt, and the molar volume ratio of the electrolyte salt to the nitrile solvent is 1 mol/L to 15 mol/L.

In some of the embodiments, the electrolyte salt satisfies at least one of the following conditions (1)-(2):
(1) the molar volume ratio of the electrolyte salt to the nitrile solvent is 2 mol/L to 12 mol/L; and
(2) the electrolyte salt includes a lithium-ion electrolyte salt.

In some of the embodiments, the components of the electrolytic solution further include a film-forming additive, and the film-forming additive includes an unsaturated cyclic carbonate-based film-forming additive;

An unsaturated ester-based film-forming additive is more beneficial to forming a dense negative electrode SEI film during the charging and discharging process of a secondary battery, further inhibiting the corrosion of lithium metal by the electrolytic solution.

In some of the embodiments, the molar ratio of the film-forming additive to the nitrile solvent is (0.05-0.5):1.

In some of the embodiments, the film-forming additive includes at least one of vinylene carbonate, vinyl ethylene carbonate, fluoroethylene carbonate, ethylene sulfate, propylene sulfate, and ethylene sulfite.

In a second aspect of the present application, provided is a secondary battery. The secondary battery includes the electrolytic solution according to the first aspect.

In some of the embodiments, the secondary battery described above includes a lithium metal secondary battery.

The secondary battery described above exhibits excellent cycle performance.

In a third aspect of the present application, provided is an electric device. The electric device includes the secondary battery according to the second aspect.

### BRIEF DESCRIPTION OF THE DRAWINGS

To more clearly illustrate the technical solutions in the embodiments of the present application, the drawings required for illustrating the embodiments of the present application are briefly described below. Apparently, the drawings in the following description illustrate merely some embodiments of the present application, and those of ordinary skills in the art may still derive other drawings from these drawings without creative efforts. In the drawings:
FIG. 1 is a schematic diagram of an embodiment of a battery cell;
FIG. 2 is an exploded view of FIG. 1;
FIG. 3 is a schematic diagram of an embodiment of a battery pack;
FIG. 4 is an exploded view of FIG. 3; and
FIG. 5 is a schematic diagram of an embodiment of an electric device in which a battery is used as a power source.

Description of the reference numerals:
1, battery pack; 2, upper case body; 3, lower case body; 4, battery cell; 41, shell body; 42, electrode assembly; 43, cover plate; 5, electric device.

### DETAILED DESCRIPTION

Embodiments of the technical solutions of the present application will be described in detail below with reference to the drawings. The following embodiments are only for illustrating the technical solutions of the present application more clearly and therefore are only exemplary and do not limit the claimed scope of the present application.

In the description of the embodiments of the present application, unless otherwise specifically defined, "plurality of" means two or more.

Reference in the present application to "embodiment" means that a particular feature, structure, or characteristic described in combination with the embodiment can be included in at least one embodiment of the present application. The references of the word in the context of the specification do not necessarily refer to the same embodiment, nor to separate or alternative embodiments exclusive of other embodiments. It will be explicitly and implicitly appreciated by those skilled in the art that the embodiments described herein can be combined with other embodiments.

In the present application, the term "alkyl" refers to a group formed by removing one hydrogen from an alkane. For example, methane loses one hydrogen to form methyl. "Alkenyl or alkynyl" refers to a group formed by removing one hydrogen from an alkene or alkyne. For example, ethylene loses one hydrogen to form ethenyl, and acetylene loses one hydrogen to form ethynyl.

In the present application, the number of carbon atoms of "alkyl with 1-3 carbon atoms" may be 1-3, including 1, 2, and 3, and non-limiting examples thereof include methyl, ethyl, and n-propyl.

In the present application, the term "substituted or unsubstituted" means that a functional group modified by the term may or may not have a substituent.

"Alkoxy" refers to a group with the structure -OR, where R is alkyl, that is, the alkyl as defined above is linked to an adjacent group via an oxygen atom. Phrases containing this term, such as "alkoxy with 1-3 carbon atoms", mean that the alkyl moiety contains 1-3 carbon atoms. Examples of alkoxy include, but are not limited to, methoxy (-O-CH₃ or -OMe) and ethoxy (-O-CH₂CH₃ or -OEt).

In the present application, the halogen group includes chlorine, fluorine, bromine, and iodine.

In the present application, R₁₁ being selected from alkane chains containing 1-4 carbon atoms means that R₁₁ is selected from residues formed by removing any three H from alkane containing 1-4 carbon atoms. In other words, in the compound represented by formula (1), any three H of alkane containing 1-4 carbon atoms are removed and substituted with R₁₂, R₁₃, and R₁₄.

In the conventional technology, acetonitrile solvents are often used as electrolytic solution additives of secondary batteries to improve the battery performance, but research shows that the acetonitrile solvents are too active and can easily react with lithium metal in the lithium metal secondary batteries to corrode the lithium metal.

In the conventional technology, in order to reduce the degree of corrosion of lithium metal by the acetonitrile solvent, a diluent is usually added into the electrolytic solution to dilute the concentration of the acetonitrile solvent.

However, research shows that by adding a diluent to the electrolytic solution to dilute the concentration of the acetonitrile solvent, the degree of corrosion of the lithium metal by the acetonitrile solvent can be reduced only in a short time, and when the acetonitrile solvent is applied to the preparation of a lithium metal battery, the acetonitrile solvent is still liable to corrode the lithium metal during the long-term cyclic use in charging and discharging process of the lithium metal battery, and the service life of the lithium metal secondary battery cannot be substantially improved.

Based on this, an exploration from the fundamental structure of acetonitrile solvents is conducted. Research shows that the type and position of substituents in the acetonitrile solvents are closely related to their redox stability.

Accordingly, in an embodiment of the present application, provided is an electrolytic solution. The components of the electrolytic solution include a nitrile solvent, and the nitrile solvent includes a compound represented by formula (1): 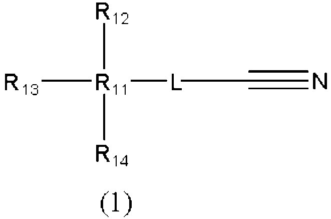
where L is selected from any one of C(R₁R₂), O, Si(R₃R₄), B(R₅), P(R₆R₇R₈), and N(R₉); R₁-R₉ are each independently selected from any one of H, halogen, substituted or unsubstituted alkyl with 1-3 carbon atoms, alkoxy with 1-3 carbon atoms, amine group, and cyano, and R₁ and R₂ are not cyano and H;
R₁₁ is an alkane chain containing 1-4 carbon atoms.
R₁₂-R₁₄ are each independently selected from any one of H, halogen, substituted or unsubstituted alkyl with 1-3 carbon atoms, alkoxy with 1-3 carbon atoms, amine group, and cyano; when L is selected from C(R₁R₂), R₁₂-R₁₄ are not cyano.

The electrolytic solution described above contains a nitrile solvent with a specific structure, and the compound represented by formula (1) contains an alkyl chain. When L is selected from O, Si(R₃R₄), B(R₅), P(R₆R₇R₈), and N(R₉), the carbon chain is separated from cyano by the groups, such that the activity of the group on the alkyl chain is reduced, and the redox stability of the nitrile solvent is improved, where O, Si, B, P, and N all can form stable connection with cyano, and Si can form a more stable electronic structure with the cyano by providing an empty orbit, thus reducing the activity of the group on the alkyl chain.

When L is selected from C(R₁R₂), that is, when cyano is directly linked to the alkyl chain, the groups of R₁ and R₂ are regulated, so that R₁ and R₂ are not cyano and H, and R₁₂-R₁₄ are not cyano. Thus, the reactivity of the carbon atom directly linked to cyano is reduced, and good redox stability is kept. As such, the compound represented by formula (1) with the specific structure described above not only exhibits good solubility for electrolyte salts but also maintains good oxidation stability. When applied to the preparation of secondary batteries, particularly lithium metal secondary batteries, it can reduce the likelihood of corrosion on the positive electrode metal, thereby enhancing the cycle performance of the battery.

In some of the embodiments, the nitrile solvent includes a compound represented by formula (1-1):

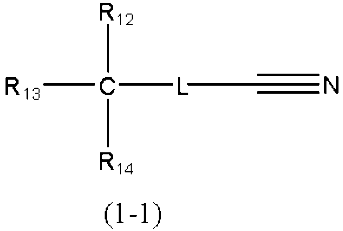

In some of the embodiments, L is selected from any one of C(R₁R₂), Si(R₃R₄), and N(R₉).

When L is selected from C(R₁R₂), Si(R₃R₄), and N(R₉), the compound represented by formula (1) can simultaneously achieve a wider melting point range, higher redox stability, and excellent lithium salt dissolving capability.

In some of the embodiments, R₁-R₉ are each independently selected from any one of H, halogen, alkyl with 1-3 carbon atoms, halogen-substituted alkyl with 1-3 carbon atoms, alkoxy with 1-3 carbon atoms, and cyano.

In some of the embodiments, R₁-R₉ are each independently selected from any one of halogen, alkyl with 1-3 carbon atoms, halogen-substituted alkyl with 1-3 carbon atoms, alkoxy with 1-3 carbon atoms, and cyano.

In some of the embodiments, R₁ and R₂ are not H at the same time, R₃ and R₄ are not H at the same time, R₆, R₇, and R₈ are not H at the same time, and R₅ and R₉ are not H.

In some of the embodiments, R₁ and R₂ are not H, R₃ and R₄ are not H, R₆, R₇, and R₈ are not H, and R₅ and R₉ are not H.

The structures of R₁-R₉ are further regulated. Compared with the case where R₁-R₉ are H, when R₁-R₉ are each independently selected from any one of halogen, alkyl with 1-3 carbon atoms, halogen-substituted alkyl with 1-3 carbon atoms, alkoxy with 1-3 carbon atoms, and cyano, the nitrile solvent exhibits higher redox stability.

In some of the embodiments, R₁-R₉ are each independently selected from any one of F, alkyl with 1-3 carbon atoms, F-substituted alkyl with 1-3 carbon atoms, alkoxy with 1-3 carbon atoms, and cyano.

In some of the embodiments, R₁-R₉ are each independently selected from any one of F, alkyl with 1-2 carbon atoms, F-substituted alkyl with 1-2 carbon atoms, alkoxy with 1-2 carbon atoms, and cyano.

In some of the embodiments, R₁-R₉ are each independently selected from any one of F, alkyl with 1-2 carbon atoms, F-substituted alkyl with 1-2 carbon atoms, alkoxy with 1-2 carbon atoms, and cyano.

In some of the embodiments, R₁-R₉ are each independently selected from any one of F, methyl, ethyl, trifluoromethyl, methoxy, ethoxy, and cyano.

In some of the embodiments, R₁₂-R₁₄ are each independently selected from any one of H, halogen, alkyl with 1-3 carbon atoms, halogen-substituted alkyl with 1-3 carbon atoms, alkoxy with 1-3 carbon atoms, and cyano.

In some of the embodiments, R₁₂-R₁₄ are each independently selected from any one of H, halogen, alkyl with 1-2 carbon atoms, halogen-substituted alkyl with 1-2 carbon atoms, alkoxy with 1-2 carbon atoms, and cyano.

In some of the embodiments, R₁₂-R₁₄ are each independently selected from any one of H, F, alkyl with 1-2 carbon atoms, F-substituted alkyl with 1-2 carbon atoms, alkoxy with 1-2 carbon atoms, and cyano.

In some of the embodiments, R₁₂-R₁₄ are each independently selected from any one of H, F, alkyl with 1-2 carbon atoms, F-substituted alkyl with 1-2 carbon atoms, alkoxy with 1-2 carbon atoms, and cyano.

In some of the embodiments, R₁₂-R₁₄ are each independently selected from any one of H, F, methyl, ethyl, trifluoromethyl, methoxy, ethoxy, and cyano.

In some of the embodiments, the nitrile solvent described above includes at least one of compounds represented by formula (1a) to formula (1f): 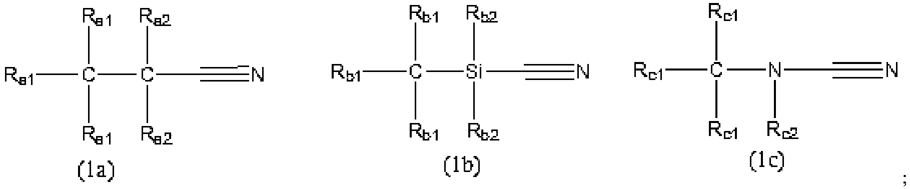 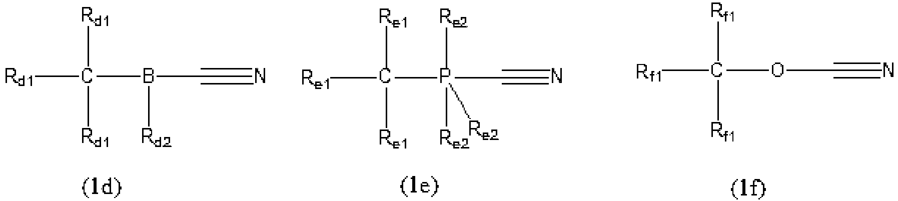
where each Rₐ₁, each R_{b1}, each R_{c1}, each R_{d1}, each Rₑ₁, and each R_{f1} are each independently selected from any one of H, halogen, alkyl with 1-3 carbon atoms, halogen-substituted alkyl with 1-3 carbon atoms, alkoxy with 1-3 carbon atoms, and cyano, and each Rₐ₁ is not cyano.
each Rₐ₂, each R_{b2}, each R_{c2}, each R_{d2}, and each Rₑ₂ are each independently selected from any one of halogen, alkyl with 1-3 carbon atoms, halogen-substituted alkyl with 1-3 carbon atoms, and alkoxy with 1-3 carbon atoms.

In some of the embodiments, each Rₐ₁, each R_{b1}, each R_{c1}, each R_{d1}, and each Rₑ₁ are each independently selected from any one of alkyl with 1-3 carbon atoms, halogen-substituted alkyl with 1-3 carbon atoms, and alkoxy with 1-3 carbon atoms.

In some of the embodiments, each Rₐ₁, each R_{b1}, each R_{c1}, each R_{d1}, and each Rₑ₁ are each independently selected from any one of alkyl with 1-2 carbon atoms, halogen-substituted alkyl with 1-2 carbon atoms, and alkoxy with 1-2 carbon atoms.

In some of the embodiments, each Rₐ₁, each R_{b1}, each R_{c1}, each R_{d1}, and each Rₑ₁ are each independently selected from any one of alkyl with 1-2 carbon atoms, F-substituted alkyl with 1-2 carbon atoms, and alkoxy with 1-2 carbon atoms.

In some of the embodiments, each Rₐ₁, each R_{b1}, each R_{c1}, each R_{d1}, and each Rₑ₁ are each independently selected from any one of methyl, ethyl, trifluoromethyl, methoxy, and ethoxy.

In some of the embodiments, at least one Rₐ₂ is alkoxy with 1-3 carbon atoms.

Research shows that in the formula (1a), when at least one Rₐ₂ is alkoxy with 1-3 carbon atoms, the nitrile solvent exhibits higher redox stability.

In some of the embodiments, at least one Rₐ₂ is alkoxy with 1-2 carbon atoms.

In some of the embodiments, at least one Rₐ₂ is methoxy or ethoxy with carbon atoms.

In some of the embodiments, at least one Rₐ₂ is alkyl with 1-3 carbon atoms.

In some of the embodiments, at least one Rₐ₂ is alkyl with 1-2 carbon atoms.

In some of the embodiments, at least one Rₐ₂ is a methyl or ethyl with carbon atoms.

In some of the embodiments, each R_{b2} is independently selected from any one of alkyl with 1-3 carbon atoms and alkoxy with 1-3 carbon atoms.

Research shows that the redox stability of the nitrile solvent can be further improved by regulating the structure of R_{b2} in the formula (1b).

In some of the embodiments, each R_{b2} is independently selected from any one of alkyl with 1-2 carbon atoms and alkoxy with 1-2 carbon atoms.

In some of the embodiments, each R_{b2} is independently selected from any one of methyl, ethyl, methoxy, and ethoxy with carbon atoms.

In some of the embodiments, each R_{c1} is independently selected from alkyl with 1-3 carbon atoms.

Research shows that the redox stability of the nitrile solvent can be further improved by regulating the structure of R_{c1} in the formula (1c).

In some of the embodiments, each R_{c1} is independently selected from alkyl with 1-2 carbon atoms.

In some of the embodiments, each R_{c1} is independently selected from methyl or ethyl.

In some of the embodiments, each R_{c2} is independently selected from any one of F, alkyl with 1-3 carbon atoms, halogen-substituted alkyl with 1-3 carbon atoms, and alkoxy with 1-3 carbon atoms.

In some of the embodiments, each R_{c2} is independently selected from any one of F, alkyl with 1-2 carbon atoms, halogen-substituted alkyl with 1-2 carbon atoms, and alkoxy with 1-2 carbon atoms.

In some of the embodiments, each R_{d1} is independently selected from alkyl with 1-3 carbon atoms.

Research shows that the redox stability of the nitrile solvent can be further improved by regulating the structure of R_{d1} in the formula (1d).

In some of the embodiments, each R_{d1} is independently selected from alkyl with 1-2 carbon atoms.

In some of the embodiments, each R_{d1} is independently selected from methyl or ethyl.

In some of the embodiments, each R_{d2} is independently selected from any one of F, alkyl with 1-3 carbon atoms, halogen-substituted alkyl with 1-3 carbon atoms, and alkoxy with 1-3 carbon atoms.

In some of the embodiments, each R_{d2} is independently selected from any one of F, alkyl with 1-2 carbon atoms, halogen-substituted alkyl with 1-2 carbon atoms, and alkoxy with 1-2 carbon atoms.

In some of the embodiments, each Rₑ₁ is independently selected from alkoxy with 1-3 carbon atoms.

In some of the embodiments, each Rₑ₁ is independently selected from alkoxy with 1-2 carbon atoms.

In some of the embodiments, each Rₑ₁ is independently selected from methoxy or ethoxy.

Research shows that the redox stability of the nitrile solvent can be further improved by regulating the structure of Rₑ₁ in the formula (1e).

In some of the embodiments, each Rₑ₂ is independently selected from any one of F, alkyl with 1-3 carbon atoms, halogen-substituted alkyl with 1-3 carbon atoms, and alkoxy with 1-3 carbon atoms.

In some of the embodiments, each Rₑ₂ is independently selected from any one of F, alkyl with 1-2 carbon atoms, halogen-substituted alkyl with 1-2 carbon atoms, and alkoxy with 1-2 carbon atoms.

In some of the embodiments, each R_{f1} is independently selected from any one of H and alkyl with 1-2 carbon atoms.

Research shows that the redox stability of the nitrile solvent can be further improved by regulating the structure of Rn in the formula (1f).

In some of the embodiments, each R_{f1} is independently selected from any one of H, methoxy, and ethoxy.

In some of the embodiments, each R_{f2} is independently selected from any one of F, alkyl with 1-3 carbon atoms, halogen-substituted alkyl with 1-3 carbon atoms, and alkoxy with 1-3 carbon atoms.

In some of the embodiments, each R_{f2} is independently selected from any one of F, alkyl with 1-2 carbon atoms, halogen-substituted alkyl with 1-2 carbon atoms, and alkoxy with 1-2 carbon atoms.

In some of the embodiments, the nitrile solvent includes at least one of compounds represented by formula (11) to formula (58):

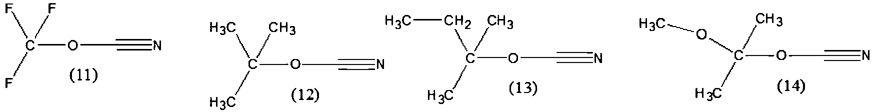

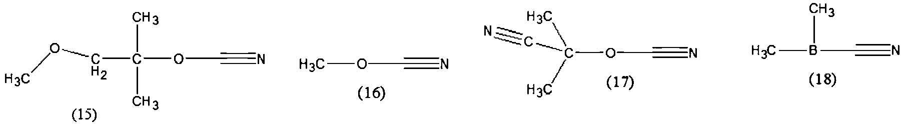

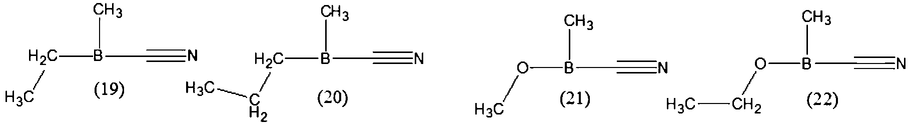

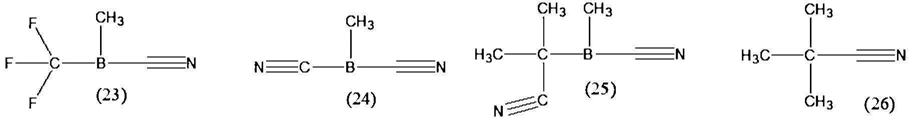

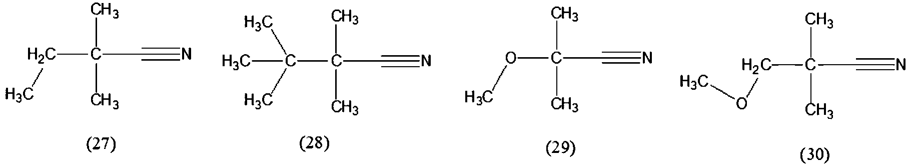

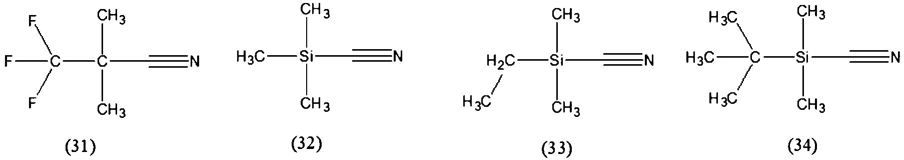

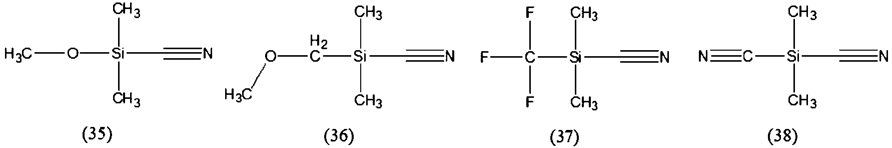

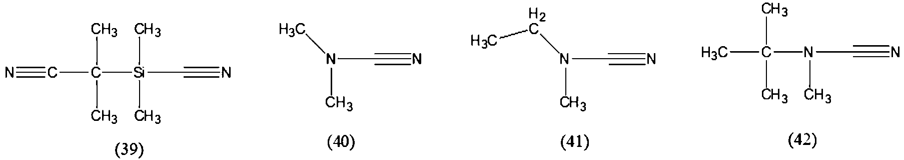

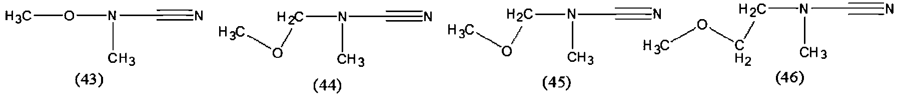

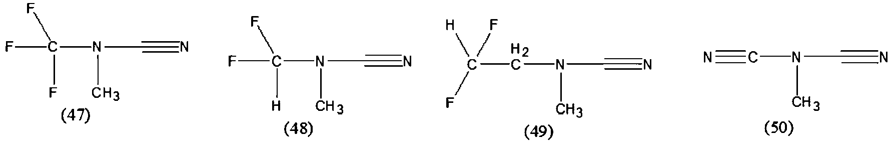

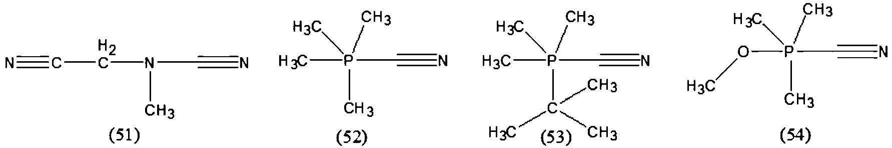

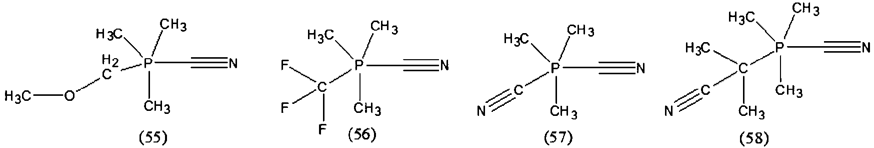

It should be noted that the above exemplified compounds are all compounds of known structure in the art or compounds obtainable according to the prior art.

In some of the embodiments, the components of the electrolytic solution further include an electrolyte salt, and the molar volume ratio of the electrolyte salt to the nitrile solvent is 1 mol/L to 15 mol/L.

Optionally, in the electrolytic solution, the molar volume ratio of the electrolyte salt to the nitrile solvent is 2 mol/L to 12 mol/L.

In the expression "1 mol/L to 15 mol/L" described above, the values include the minimum value and the maximum value of the range and each value between the minimum value and the maximum value, and specific examples include, but are not limited to, the point values in the embodiments and the following point values: 1 mol/L, 2 mol/L, 3 mol/L, 4 mol/L, 5 mol/L, 6 mol/L, 7 mol/L, 8 mol/L, 9 mol/L, 10 mol/L, 11 mol/L, 12 mol/L, 13 mol/L, 14 mol/L, and 15 mol/L, or ranges formed by any two of the above values, such as 1 mol/L to 15 mol/L, 1 mol/L to 10 mol/L, 1 mol/L to 5 mol/L, 2 mol/L to 15 mol/L, 3 mol/L to 15 mol/L, 4 mol/L to 15 mol/L, 5 mol/L to 15 mol/L, 6 mol/L to 15 mol/L, 7 mol/L to 15 mol/L, 8 mol/L to 15 mol/L, 9 mol/L to 15 mol/L, 10 mol/L to 15 mol/L, 3 mol/L to 10 mol/L, 4 mol/L to 10 mol/L, and 5 mol/L to 10 mol/L.

In some of the embodiments, the components of the electrolytic solution further include a film-forming additive.

In some of the embodiments, the film-forming additive includes an unsaturated ester-based film-forming additive; further, the unsaturated ester-based film-forming additive includes at least one of an unsaturated cyclic carbonate-based film-forming additive, an unsaturated carboxylate-based film-forming additive, and an unsaturated sulphate-based film-forming additive.

In some of the embodiments, the film-forming additive includes an unsaturated cyclic carbonate-based film-forming additive.

An unsaturated cyclic carbonate-based film-forming additive is more beneficial to forming a dense negative electrode SEI film during the charging and discharging process of a secondary battery, further inhibiting the corrosion of lithium metal by the electrolytic solution.

It should be noted that the unsaturated cyclic carbonate-based film-forming additive refers to a film-forming additive in which the carbonate ring contains a carbon-carbon double bond.

Specifically, the unsaturated cyclic carbonate-based film-forming additive has the following structure: 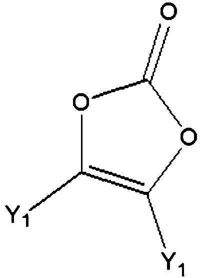 where Y₁ and Y₂ are each independently selected from any one of H, alkyl with 1-5 carbon atoms, and alkenyl with 2-5 carbon atoms.

In some of the embodiments, Y₁ and Y₂ are each independently selected from any one of H, chain alkyl with 1-5 carbon atoms, and chain alkenyl with 2-5 carbon atoms.

In some of the embodiments, Y₁ and Y₂ are each independently selected from any one of H, alkyl with 1-3 carbon atoms, and alkenyl with 2-3 carbon atoms.

In some of the embodiments, Y₁ and Y₂ are each independently selected from any one of H, chain alkyl with 1-3 carbon atoms, and chain alkenyl with 2-3 carbon atoms.

In some of the embodiments, Y₁ and Y₂ are identical or different.

In some of the embodiments, the film-forming additive includes at least one of vinylene carbonate, vinyl ethylene carbonate, fluoroethylene carbonate, ethylene sulfate, propylene sulfate, and ethylene sulfite.

In some of the embodiments, the film-forming additive includes vinylene carbonate.

In some of the embodiments, the molar ratio of the film-forming additive to the nitrile solvent is (0.05-0.5):1.

In the expression "(0.05-0.5):1" described above, the values include the minimum value and the maximum value of the range and each value between the minimum value and the maximum value, and specific examples include, but are not limited to, the point values in the embodiments and the following point values: 0.5:1, 0.4:1, 0.3:1, 0.2:1, 0.1:1, 0.09:1, 0.09:1, 0.08:1, 0.07:1, 0.06:1, and 0.05:1, or ranges formed by any two of the above values, such as (0.08-0.5):1, (0.09-0.5):1, (0.1-0.5):1, (0.2-0.5):1, (0.3-0.5):1, (0.3-0.4):1, (0.1-0.4):1, (0.1-0.3):1, and (0.2-0.4):1.

In some of the embodiments, the components of the electrolytic solution further include a diluent.

The diluent is beneficial to reducing the viscosity of the electrolytic solution and improving the ionic conductivity.

In some of the embodiments, the molar ratio of the diluent to the nitrile solvent is (0.5-5):1.

By regulating the molar ratio of the diluent to the nitrile solvent, the reduction stability of the electrolytic solution is further improved while maintaining the good ionic conductivity of the electrolytic solution.

In the expression "(0.5-5):1" described above, the values include the minimum value and the maximum value of the range and each value between the minimum value and the maximum value, and specific examples include, but are not limited to, the point values in the embodiments and the following point values: 5:1, 4.5:1, 4:1, 3.5:1, 3:1, 2.5:1, 2:1, 1.5:1, 1:1, and 0.5:1, or ranges formed by any two of the above values, such as (1-5):1, (2-5):1, (3-5):1, (2-4):1, and (3-4):1.

In some of the embodiments, the diluent includes at least one of a fluoroether-containing compound, an aromatic hydrocarbon solvent, and a fluoro-containing aromatic solvent.

In some of the embodiments, the diluent includes at least one of 1,1,2,2-tetrafluoroethyl-2,2,3,3-tetrafluoropropyl ether, 1,1,2,2-tetrafluoroethyl-2,2,2-trifluoroethyl ether, 1-(1,1,2,2-tetrafluoroethoxy)propane, 1,1,2,2-tetrafluoroethyl ethyl ether, benzene, and trifluoromethoxybenzene.

In some of the embodiments, the electrolyte salt may be selected from electrolyte salts commonly used in the art, such as a lithium-ion electrolyte salt, a sodium-ion electrolyte salt, and a potassium-ion electrolyte salt.

In some of the embodiments, the electrolyte salt described above includes a lithium-ion electrolyte salt.

As an example, the lithium-ion electrolyte salt includes, but is not limited to, one or more of lithium hexafluorophosphate (LiPF₆), lithium tetrafluoroborate (LiBF₄), lithium perchlorate (LiClO₄), lithium hexafluoroarsenate (LiAsF₆), lithium bis(fluorosulfonyl)imide (LiFSI), lithium bis(trifluoromethanesulfonyl)imide (LiTFSI), lithium trifluoromethanesulfonate (LiTFS), lithium difluoro(oxalato)borate (LiDFOB), lithium bis(oxalato)borate (LiBOB), lithium difluorophosphate (LiPO₂F₂), lithium difluorobis(oxalato)phosphate (LiDFOP), and lithium tetrafluoro(oxalato)phosphate (LiTFOP).

As an example, the sodium-ion electrolyte salt includes, but is not limited to, one or more of sodium hexafluorophosphate, sodium difluoro(oxalato)borate, sodium tetrafluoroborate, sodium bis(oxalato)borate, sodium perchlorate, sodium hexafluoroarsenate, sodium bis(fluorosulfonyl)imide, sodium trifluoromethanesulfonate, and sodium bis(trifluoromethylsulfonyl)imide.

In an embodiment of the present application, provided is a secondary battery. The secondary battery includes the electrolytic solution according to the first aspect.

In some of the embodiments, the secondary battery further includes a positive electrode plate, a negative electrode plate, and a separator.

Positive electrode plate: the positive electrode plate includes a current collector and a positive electrode active layer loaded on the surface of the current collector.

The components of the positive electrode active layer include a positive electrode active material. The positive electrode active material may be selected from positive electrode active materials commonly used in the art, including but not limited to: a positive electrode active material for lithium-ion batteries, a positive electrode active material for sodium-ion batteries, and a positive electrode active material for potassium-ion batteries.

The positive electrode active material for lithium-ion batteries, the positive electrode active material for sodium-ion batteries, and the positive electrode active material for potassium-ion batteries are hereinafter referred to simply as a lithium-ion active material, a sodium-ion active material, and a potassium-ion active material, respectively.

Further, as an example, the lithium-ion active material may include at least one of the following materials: a lithium-containing phosphate with an olivine structure, a lithium transition metal oxide, and respective modified compounds thereof. However, the present application is not limited to these materials, and other conventional materials that can be used as positive electrode active materials for batteries may also be used. These positive electrode active materials may be used alone or in combination of two or more. Examples of the lithium transition metal oxide may include, but are not limited to, at least one of a lithium cobalt oxide (e.g., LiCoO₂), a lithium nickel oxide (e.g., LiNiO₂), a lithium manganese oxide (e.g., LiMnO₂ and LiMn₂O₄), a lithium nickel cobalt oxide, a lithium manganese cobalt oxide, a lithium nickel manganese oxide, a lithium nickel cobalt manganese oxide (e.g., LiNi_{1/3}Co_{1/3}Mn_{1/3}O₂ (also referred to as NCM333), LiNi_{0.5}Co_{0.2}Mn_{0.3}O₂ (also referred to as NCM523), LiNi_{0.5}Co_{0.25}Mn_{0.25}O₂ (also referred to as NCM211), LiNi_{0.6}Co_{0.2}Mn_{0.2}O₂ (also referred to as NCM622), LiNi_{0.8}Co_{0.1}Mn_{0.1}O₂ (also referred to as NCM811)), a lithium nickel cobalt aluminum oxide (e.g., LiNi_{0.8}Co_{0.15}Al_{0.05}O₂), and modified compounds thereof. Examples of the lithium-containing phosphate with an olivine structure may include, but are not limited to, at least one of lithium iron phosphate (such as LiFePO₄ (also referred to as LFP)), lithium manganese phosphate (such as LiMnPO₄), and lithium manganese iron phosphate. In any embodiment of the present application, the molecular formula of the lithium-ion active material is LiFeₓMn₍₁₋ₓ₎PO₄, where x is any number of 0 to 1.

It can be understood that when x is 0, LiFeₓMn₍₁₋ₓ₎PO₄ is LiMnPO₄ lithium manganese phosphate, and when x is 1, LiFeₓMn₍₁₋ₓ₎PO₄ is LiFePO₄ lithium iron phosphate (LFP).

It should be noted that the lithium content in the above exemplified positive electrode materials refers to the content when the positive electrode material is not used; during the use of the battery, the battery will be charged and discharged repeatedly, and Li in the positive electrode active material changes during the charging and discharging process, that is, the molar subscript of Li in the positive electrode active material in the battery product does not always remain at 1, and it varies; further, the variation range may be (0-1.2).

For example, LiFeₓMn₍₁₋ₓ₎PO₄ may be further represented as Li_{y}FeₓMn₍₁₋ₓ₎PO₄, where y is 0-1.1.

For example, for the ternary material Li_{y}(NiₐCo_{b}Mn_{c})_{1-d}M_{d}O₂₋ₓAₓ, y is 0.2-1.2, a + b + c = 1, 0 ≤ d ≤ 1, 0 ≤ x < 2, M is one or more of Zr, Sr, B, Ti, Mg, Sn, and Al, and A is one or more of S, N, F, Cl, Br, and I.

During the charging and discharging process of a battery, the deintercalation and consumption of Li ions occur. The molar content of Li varies when the battery is discharged to different states. The above constraints on y encompass the molar content of Li across various charging and discharging states. Further, the battery voltage typically ranges between 2-5 V.

As an example, the sodium-ion active material may include at least one of the following materials: at least one of a sodium transition metal oxide, a polyanionic compound, and a Prussian blue compound. However, the present application is not limited to these materials, and other conventional and well-known materials that can be used as positive electrode active materials for sodium batteries may also be used.

As an optional technical solution of the present application, in the sodium transition metal oxide, the transition metal at least includes at least one of Mn, Fe, Ni, Co, Cr, Cu, Ti, Zn, V, Zr, and Ce. The sodium transition metal oxide is, e.g., NaₓMO₂, where M at least includes one or more of Ti, V, Mn, Co, Ni, Fe, Cr, and Cu, and 0 < x ≤ 1.

As an optional technical solution of the present application, the polyanionic compound may be a class of compounds having sodium ions, transition metal ions, and tetrahedral (YO₄)ⁿ⁻ anion units. The transition metal at least includes at least one of Mn, Fe, Ni, Co, Cr, Cu, Ti, Zn, V, Zr, and Ce; Y at least includes at least one of P, S, and Si; n represents the valence state of (YO₄)ⁿ⁻.

The polyanionic compound may also be a class of compounds having sodium ions, transition metal ions, tetrahedral (YO₄)ⁿ⁻ anion units, and halogen anions. The transition metal at least includes at least one of Mn, Fe, Ni, Co, Cr, Cu, Ti, Zn, V, Zr, and Ce; Y at least includes at least one of P, S, and Si, and n represents the valence state of (YO₄)ⁿ⁻; the halogen may be at least one of F, Cl, and Br.

The polyanionic compound may also be a class of compounds having sodium ions, tetrahedral (YO₄)ⁿ⁻ anion units, polyhedral units (ZO_{y})^{m+}, and optional halogen anions. Y at least includes at least one of P, S, and Si, and n represents the valence state of (YO₄)ⁿ⁻; Z represents a transition metal and at least includes at least one of Mn, Fe, Ni, Co, Cr, Cu, Ti, Zn, V, Zr, and Ce, and m represents the valence state of (ZO_{y})^{m+}; the halogen may be at least one of F, Cl, and Br.

The polyanionic compound is, e.g., at least one of NaFePO₄, Na₃V₂(PO₄)₃ (sodium vanadium phosphate, referred to as NVP), Na₄Fe₃(PO₄)₂(P₂O₇) (NFPP), NaM1PO₄F, and Na₃(VO_{y})₂(PO₄)₂F_{(3-2y)}.

M1 is at least one of V, Fe, Mn, and Ni, and 0 ≤ y ≤ 1.

The Prussian blue compound may be a class of compounds having sodium ions, transition metal ions, and cyanide ions (CN). The transition metal at least includes at least one of Mn, Fe, Ni, Co, Cr, Cu, Ti, Zn, V, Zr, and Ce. The Prussian blue compound is, e.g., NaₐM2_{b}M3_{c}(CN)₆, where M2 and M3 are each independently selected from at least one of Ni, Cu, Fe, Mn, Co, and Zn, 0 < a ≤ 2, 0 < b < 1, and 0 < c < 1.

In any embodiment of the present application, in the positive electrode active layer, the mass percentage of the positive electrode active material is 70%-99.8%.

In any embodiment of the present application, the components of the positive electrode active layer further include a conductive agent and a binder.

The conductive agent may be any conductive agent commonly used in the art, including but not limited to at least one of graphite, carbon nanotubes, nanofibers, carbon black, and graphene. Specifically, the conductive agent may be selected from at least one of SP, KS-6, acetylene black, branched Ketjen black ECP, SFG-6, vapor grown carbon fiber VGCF, carbon nanotubes CNTs, graphene, and a composite conductive agent thereof.

The binder of the binder may be at least one of polyvinylidene difluoride (PVDF), polytetrafluoroethylene (PTFE), vinylidene fluoride-tetrafluoroethylene-propylene terpolymer, vinylidene fluoride-hexafluoropropylene-tetrafluoroethylene terpolymer, tetrafluoroethylene-hexafluoropropylene copolymer, hydrogenated nitrile-butadiene rubber, styrene-butadiene rubber (SBR), polyacrylic acid (PAA), sodium polyacrylate (PAAS), polyacrylamide (PAM), polyvinyl alcohol (PVA), sodium alginate (SA), polymethacrylic acid (PMAA), carboxymethyl chitosan (CMCS), and fluorinated acrylate resin.

Optionally, in the positive electrode active layer, the mass percentage of the conductive agent is 1%-20%.

Optionally, in the positive electrode active layer, the mass percentage of the binder is 1%-10%.

In some of the embodiments, the thickness of the positive electrode active layer described above is 30 µm to 200 µm.

In any embodiment of the present application, the current collector may be a metal foil or a composite current collector. For example, as the metal foil, an aluminum foil may be used. The composite current collector may include a polymer material substrate and a metal layer formed on at least one surface of the polymer material substrate. The composite current collector may be fabricated by forming a metal material on a polymer material substrate.

In some of the embodiments, the metal material is selected from any one of aluminum, an aluminum alloy, nickel, a nickel alloy, titanium, a titanium alloy, silver, and a silver alloy.

In some of the embodiments, the polymer material substrate includes at least one of polypropylene (PP), polyethylene terephthalate (PET), polybutylene terephthalate (PBT), polystyrene (PS), and polyethylene (PE).

In any embodiment of the present application, the positive electrode plate may be prepared in the following manner: dispersing the components described above for preparing the positive electrode plate in an organic solvent to form a positive electrode slurry; and coating a current collector with the positive electrode slurry, and performing drying, cold pressing, and other processes, such that the positive electrode plate can be obtained.

In some of the embodiments, the solid content of the positive electrode slurry is 40 wt% to 80 wt%, and the viscosity at 25 °C is adjusted to be 5000 mPa·s to 25000 mPa·s.

In some of the embodiments, the organic solvent includes, but is not limited to, N-methylpyrrolidone.

In some of the embodiments, the positive electrode active material contained in the positive electrode plate has an areal density of 0.018 g/cm² to 0.05 g/cm².

The areal density of the positive electrode active material = the mass of the positive electrode active material/the area of the positive electrode plate.

Negative electrode plate: the negative electrode plate may be a negative electrode plate system of various types of secondary batteries commonly used in the art. The following takes lithium-ion secondary batteries and lithium metal secondary batteries as examples for illustration, but the batteries are not limited to the following types.

In some of the embodiments, the secondary battery is a lithium metal secondary battery, and the negative electrode plate may be a well-known negative electrode plate that may be used for the lithium metal batteries in the art.

In some embodiments, the negative electrode plate is directly a lithium-containing metal plate.

In another embodiment, the negative electrode plate includes a lithium-containing metal layer and a conductive layer arranged in a stacked manner.

Further, the lithium-containing technology in the lithium-containing metal plate and the lithium-containing metal layer may be lithium metal, or may be an alloy formed by lithium metal and other metals or nonmetal elements.

Further, the other metals include at least one of tin (Sn), zinc (Zn), aluminum (Al), magnesium (Mg), silver (Ag), gold (Au), gallium (Ga), indium (In), and foil (Pt); the nonmetal elements include at least one of boron (B), carbon (C), and silicon (Si).

In some of the embodiments, the conductive layer may be a copper foil.

In any embodiment of the present application, the negative electrode plate described above may be prepared in the following manner: directly pressing the lithium-containing metal plate to obtain the negative electrode plate, or laminating the lithium-containing metal layer and the conductive layer described above to obtain the negative electrode plate.

In some of the embodiments, the secondary battery is a lithium-ion secondary battery, and the negative electrode plate may be a well-known negative electrode plate that may be used for the lithium-ion secondary batteries in the art.

In some of the embodiments, the negative electrode plate includes a current collector and a negative electrode active layer loaded on the surface of the current collector.

The components of the negative electrode active layer include a negative electrode active material.

The negative electrode active material described above may be any negative electrode active material commonly used in the present application.

In any embodiment of the present application, the negative electrode active material described above includes at least one of mesocarbon microbeads, graphite, glassy carbon, carbon nanotubes, carbon-carbon composite materials, carbon fibers, hard carbon, soft carbon, silicon-based materials, tin-based materials, magnesium-based materials, and iron-based materials.

Optionally, specific examples of the negative electrode active material described above include, but are not limited to, at least one of mesocarbon microbeads, natural graphite, artificial graphite, graphene, glassy carbon, carbon nanotubes, carbon fibers, hard carbon, soft carbon, iron oxide, tin oxide, silicon oxide, magnesium oxide, silicon-carbon composites, lithium metal, and lithium metal alloys.

In any embodiment of the present application, the mass percentage of the negative electrode active material described above in the negative electrode active layer is 70%-100%.

In any embodiment of the present application, the components of the negative electrode active layer described above further include a negative electrode conductive agent and a negative electrode binder.

In any embodiment of the present application, the negative electrode conductive agent described above may be a conductive material commonly used in the art, including but not limited to at least one of graphite, carbon nanotubes, nanofibers, carbon black, and graphene. Specifically, the negative electrode conductive agent may be selected from at least one of SP, KS-6, acetylene black, branched Ketjen black ECP, SFG-6, vapor grown carbon fiber VGCF, carbon nanotubes CNTs, graphene, and a composite conductive agent thereof.

The weight ratio of the negative electrode conductive agent in the negative electrode active layer is 0 wt% to 20 wt% based on the total weight of the negative electrode active layer.

The negative electrode binder described above may be a binder commonly used in the art and may be selected from at least one of styrene-butadiene rubber (SBR), polyacrylic acid (PAA), sodium polyacrylate (PAAS), polyacrylamide (PAM), polyvinyl alcohol (PVA), sodium alginate (SA), polymethacrylic acid (PMAA), and carboxymethyl chitosan (CMCS).

The weight ratio of the negative electrode binder in the negative electrode active layer is 0 wt% to 30 wt% based on the total weight of the negative electrode active layer.

In any embodiment of the present application, the negative electrode active layer further optionally includes other auxiliary agents, for example, a thickener, such as sodium carboxymethylcellulose (CMC-Na). The weight ratio of the other auxiliary agents in the negative electrode active layer is 0 wt% to 15 wt% based on the total weight of the negative electrode active layer.

In any embodiment of the present application, the current collector in the negative electrode plate may be a metal foil or a composite current collector. For example, as the metal foil, a copper foil may be used.

The composite current collector may include a polymer material substrate and a metal layer formed on at least one surface of the polymer material substrate. The composite current collector may be fabricated by forming a metal material on a polymer material substrate.

In some of the embodiments, the metal material is selected from any one of aluminum, an aluminum alloy, nickel, a nickel alloy, titanium, a titanium alloy, silver, and a silver alloy.

In some of the embodiments, the polymer material substrate includes at least one of polypropylene (PP), polyethylene terephthalate (PET), polybutylene terephthalate (PBT), polystyrene (PS), and polyethylene (PE).

In any embodiment of the present application, the negative electrode plate may be prepared in the following manner: dispersing the components described above for preparing the negative electrode plate, such as the negative electrode active material, the conductive agent, the binder, and any other components, in a solvent to form a negative electrode slurry; and coating the negative electrode current collector with the negative electrode slurry, and performing drying, cold pressing, and other processes, such that the negative electrode plate can be obtained.

In some of the embodiments, the solvent described above includes, but is not limited to, water.

In some of the embodiments, the solid content of the negative electrode slurry is 30 wt% to 70 wt%, and the viscosity at 25 °C is adjusted to 2000 mPa·s to 10000 mPa·s.

In some of the embodiments, the negative electrode active material contained in the negative electrode plate has an areal density of 0.005 g/cm² to 0.03 g/cm².

The areal density of the negative electrode active material = the mass of the negative electrode active material/the area of the negative electrode plate.

Separator: the separator is disposed between the positive electrode plate and the negative electrode plate.

The present application does not particularly limit the type of the separator, and any porous-structure separator known to have good chemical stability and mechanical stability may be selected and used.

In some of the embodiments, the separator may be made of a material selected from at least one of glass fiber, nonwoven fabric, polyethylene, polypropylene, and polyvinylidene difluoride.

Optionally, the separator may be a single-layer film or a multi-layer composite film. Further, when the separator is a multi-layer composite film, the materials of the layers may be the same or different.

In some of the embodiments, the thickness of the separator is 2 µm to 15 µm; optionally, the thickness of the separator is 2 µm to 13 µm.

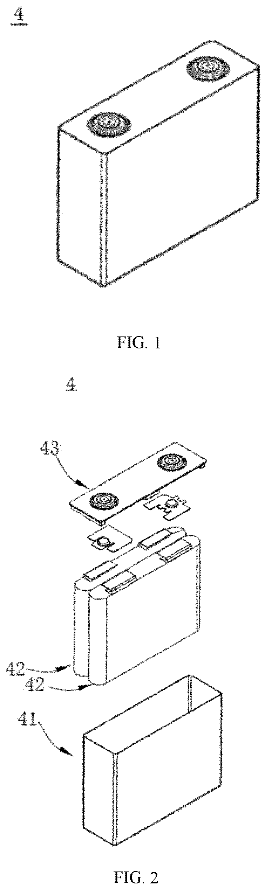

The shape of the secondary battery of the present application may be cylindrical, prismatic, or any other shape. For example, FIG. 1 shows a battery cell 4 of a secondary battery having a prismatic structure as one example.

In some embodiments, referring to FIG. 2, the shell may include a shell body 41 and a cover plate 43. The shell body 41 may include a bottom plate and side plates connected to the bottom plate, and the bottom plate and the side plates define, in an enclosing manner, an accommodating cavity. The shell body 41 is provided with an opening in communication with the accommodating cavity, and the cover plate 43 is capable of lidding the opening to close the accommodating cavity.

The positive electrode plate, the negative electrode plate, and the separator may be subjected to a winding process or a stacking process to form an electrode assembly 42. The electrode assembly 42 is packaged in the accommodating cavity. The electrolytic solution is infiltrated into the electrode assembly 42. The number of electrode assemblies 42 included in the battery cell 4 may be one or more and may be adjusted as needed.

The secondary battery includes one or more battery cells 4.

The secondary battery may be a battery module or a battery pack; the battery module or the battery pack includes at least one battery cell. The number of the battery cells included in the battery module may be one or more, and those skilled in the art may select an appropriate number according to the application and capacity of the battery module.

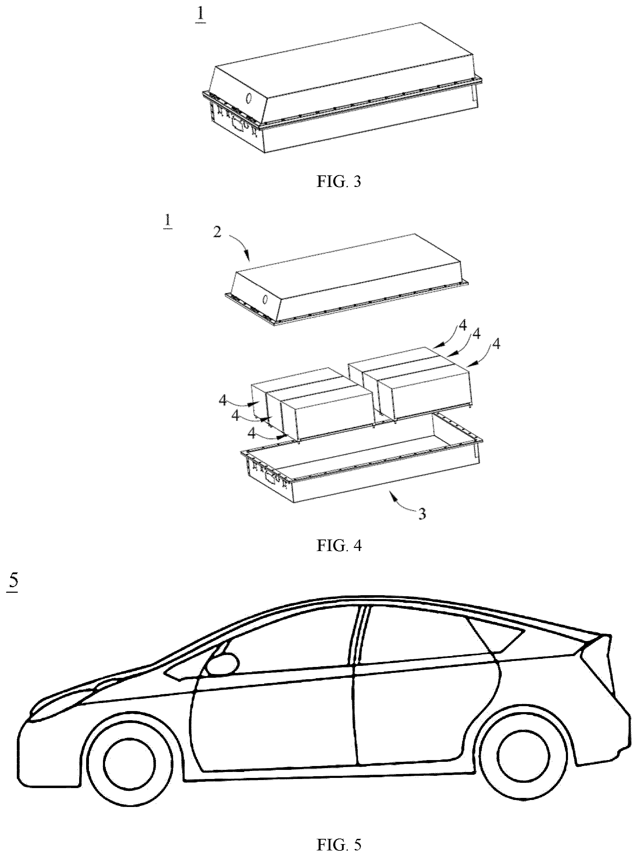

FIG. 3 and FIG. 4 show a battery pack 1 as one example. The battery pack 1 includes a battery case and one or a plurality of battery cells 4 disposed in the battery case. The battery case includes an upper case body 2 and a lower case body 3. The upper case body 2 is capable of lidding the lower case body 3 to form a closed space for the battery cells 4.

The plurality of battery cells 4 may be disposed in any manner in the battery case.

The present application further provides an electric device. The electric device includes the secondary battery described above.

Further, in the electric device described above, the secondary battery may be in the form of a battery cell, or may be in the form of a battery pack resulting from further assembling.

The batteries described above or the battery pack assembled therefrom may be used as a power source for the electric device and may also be used as an energy storage unit for the electric device.

The electric device described above may be, but is not limited to, a mobile device, an electric vehicle, an electric train, a ship, a satellite, an energy storage system, and the like.

In some of the embodiments, the mobile device may be a mobile phone, a notebook computer, or the like.

In some of the embodiments, the electric vehicle includes, but is not limited to, a pure electric vehicle, a hybrid electric vehicle, a plug-in hybrid electric vehicle, an electric bicycle, an electric scooter, an electric golf cart, an electric truck, and the like.

FIG. 5 shows an electric device 5 as one example. The electric device 5 is a pure electric vehicle, a hybrid electric vehicle, a plug-in hybrid electric vehicle, or the like. To meet the requirements of the electric device 5 for high power and high energy density of the secondary battery, the battery pack may be used.

As another example, the electric device may be a mobile phone, a tablet computer, a notebook computer, or the like. The device is generally required to be light and thin, and a battery can thus be used as a power source.

The present disclosure will be described below in conjunction with specific examples. However, the present disclosure is not limited to the following examples. It should be understood that the appended claims encompass the scope of the present disclosure, and under the guidance of the concept of the present disclosure, those skilled in the art should recognize that certain changes of the examples of the present disclosure shall fall within the spirit and scope of the claims of the present disclosure.

The specific examples are as follows.

### Example 1

### S1. Preparation of lithium metal secondary battery

### (1) Preparation of electrolytic solution:

A nitrile solvent, a diluent, and a film-forming agent were mixed uniformly according to a certain proportion to obtain a transparent solution. A certain amount of lithium bis(fluorosulfonyl)imide salt was added to 10 mL of the mixed solvent above, and the mixture was fully stirred to obtain an electrolytic solution for later use.

Components and mass percentages thereof in the electrolytic solution were separated and analyzed by combining gas chromatography and ion chromatography, and a nuclear magnetic resonance test was also performed. Thus, the structure of the nitrile solvent in the electrolytic solution, the molar volume ratio of the lithium bis(fluorosulfonyl)imide salt to the nitrile solvent M1, the molar ratio of the diluent to the nitrile solvent M2, and the molar ratio of the film-forming agent to the nitrile solvent M3 could be confirmed. The details are shown in Table 1.

The types of nitrile solvents are specified in Table 1.

### (2) Preparation of negative electrode plate

A 50 µm lithium foil was roller-pressed on the surface of a 12 µm copper foil, and then the laminate was cut into rectangular pieces of 41 mm×51 mm for use as negative electrode plates.

### (3) Preparation of positive electrode plate

Preparation of positive electrode: a positive electrode active material lithium nickel cobalt manganate (NCM811), a conductive agent acetylene black, and a binder PVDF were mixed according to a mass ratio of 98:1:1, a solvent NMP was added, and the mixture was stirred until the system was uniform, thus obtaining a positive electrode slurry;
the positive electrode slurry was uniformly applied on both sides of the positive electrode current collector aluminum foil, and then the aluminum foil was aired at room temperature, transferred to an oven for further drying, and then cut into rectangular pieces of 40 mm×50 mm for use as positive electrode plates, where the areal capacity of the positive electrode plate was 4 mAh/cm².

(4) Separator: a polyethylene porous film was selected.

(5) Assembly of lithium metal secondary battery: a pre-cut positive electrode plate was matched with a pre-cut negative electrode plate, the aforementioned separator was placed therebetween to isolate the positive and negative electrodes, and then they were wrapped in an aluminum-plastic film pouch to form a stacked dry cell; 0.3 g of the aforementioned prepared electrolytic solution was injected, and the aluminum-plastic film pouch was packaged under vacuum through hot pressing, and then left to stand at room temperature for 6 h to obtain a lithium metal secondary battery with a rated capacity of 140 mAh.

S2. Performance test of lithium metal secondary battery:
At 25 °C, the lithium metal secondary battery was subjected to charge-discharge cycling at a rate of 0.5C (i.e., 70 mA), with cut-off voltages for charging and discharging set at 4.3 V and 2.8 V, respectively. One full cycle consisted of a charge followed by a discharge.

When the discharge capacity of the lithium metal secondary battery decayed to 80% of the first-cycle discharge capacity, the service life of the battery was considered to end, and the number of charge-discharge cycles completed was recorded.

The specific results are shown in Table 1.

### Examples 2-18

Examples 2-18 are substantially the same as Example 1, except that the type of the nitrile solvent in the preparation of the electrolytic solution was different from that in Example 1, and the details are shown in Table 1.

The other steps and conditions were the same as those in Example 1. The test results are shown in Table 1.

### Examples 19-24

Examples 19-24 are substantially the same as Example 10, except that the molar volume ratio of lithium bis(fluorosulfonyl)imide to the nitrile solvent in the preparation of the electrolytic solution was regulated to be different from that in Example 10, and the details are shown in Table 1.

The other steps and conditions were the same as those in Example 10. The test results are shown in Table 1.

### Examples 25-32

Examples 25-32 are substantially the same as Example 10, except that the amount or type of the diluent and the amount or type of the film-forming agent in the preparation of the electrolytic solution were different from those in Example 10, and the details are shown in Table 1.

The other steps and conditions were the same as those in Example 10. The test results are shown in Table 1.

### Comparative Example 1

Comparative Example 1 is substantially the same as Example 1 except that the type of the nitrile solvent in the preparation of the electrolytic solution was different from that in Example 1, with the nitrile solvent in Comparative Example 1 being acetonitrile, and the details are shown in Table 1.

The other steps and conditions were the same as those in Example 1. The test results are shown in Table 1.

### Comparative Example 2

Comparative Example 2 is substantially the same as Example 1, except that the type of the nitrile solvent in the preparation of the electrolytic solution was different from that in Example 1, with the nitrile solvent in Comparative Example 2 being acetonitrile, and the amount added was different from that in Example 1, and the details are shown in Table 1.

The other steps and conditions were the same as those in Example 1. The test results are shown in Table 1.

### Comparative Example 3

Comparative Example 3 is substantially the same as Example 1, except that the type of the nitrile solvent in the preparation of the electrolytic solution was different from that in Example 1, with the nitrile solvent in Comparative Example 3 being adiponitrile as shown in chemical formula (A), and the details are shown in Table 1.

The other steps and conditions were the same as those in Example 1. The test results are shown in Table 1.

The relevant parameters and performance results for all the examples and comparative examples are shown in Table 1. The structures of the nitrile solvents used in the examples and comparative examples are shown below.

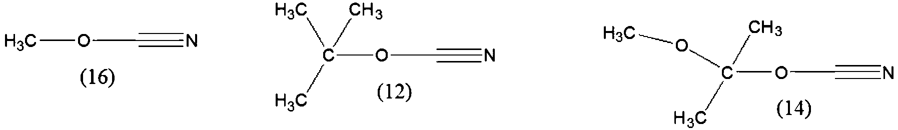

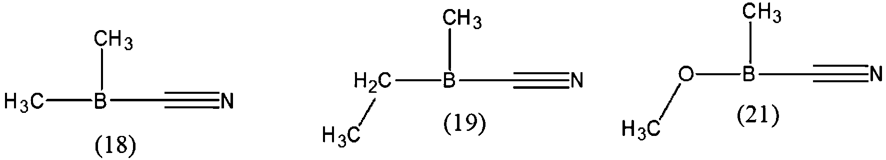

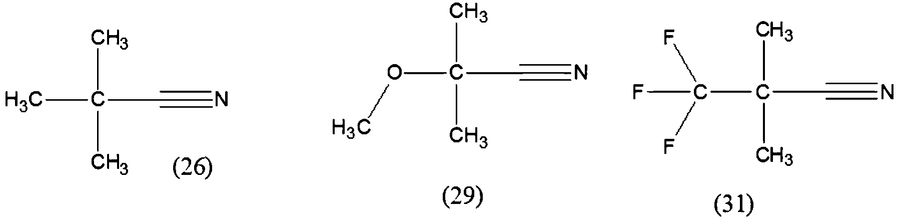

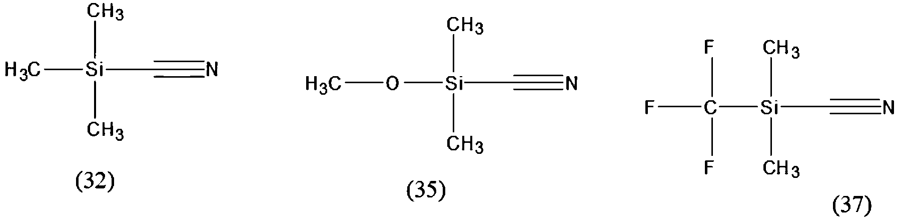

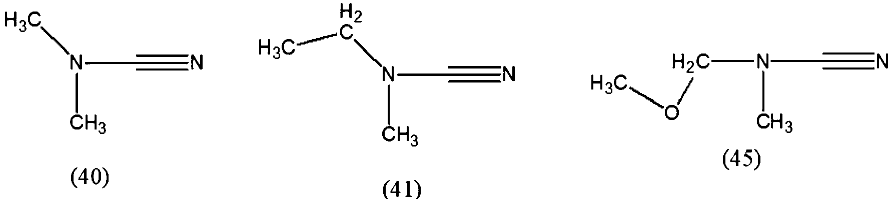

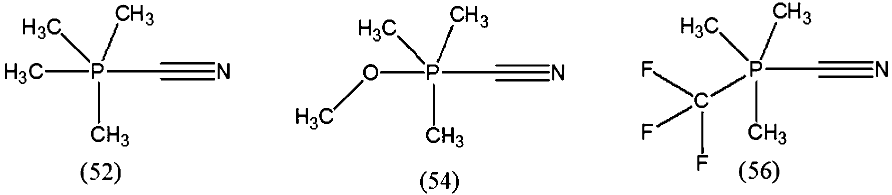

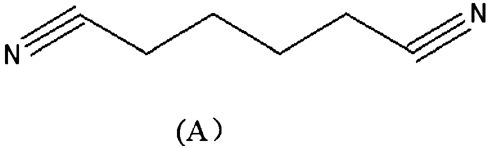

**Table 1**

| No. | M1 (mol/L) | Nitrile solvent | Diluent | M2 | Film-forming agent | M3 | Number of cycles |
|---|---|---|---|---|---|---|---|
| Example 1 | 2 | (16) | / | 0 | / | 0 | 50 |
| Example 2 | 2 | (12) | / | 0 | / | 0 | 34 |
| Example 3 | 2 | (14) | / | 0 | / | 0 | 29 |
| Example 4 | 2 | (18) | / | 0 | / | 0 | 58 |
| Example 5 | 2 | (19) | / | 0 | / | 0 | 42 |
| Example 6 | 2 | (21) | / | 0 | / | 0 | 32 |
| Example 7 | 2 | (26) | / | 0 | / | 0 | 38 |
| Example 8 | 2 | (29) | / | 0 | / | 0 | 42 |
| Example 9 | 2 | (31) | / | 0 | / | 0 | 36 |
| Example 10 | 2 | (32) | / | 0 | / | 0 | 59 |
| Example 11 | 2 | (35) | / | 0 | / | 0 | 66 |
| Example 12 | 2 | (37) | / | 0 | / | 0 | 42 |
| Example 13 | 2 | (40) | / | 0 | / | 0 | 63 |
| Example 14 | 2 | (41) | / | 0 | / | 0 | 48 |
| Example 15 | 2 | (45) | / | 0 | / | 0 | 39 |
| Example 16 | 2 | (52) | / | 0 | / | 0 | 36 |
| Example 17 | 2 | (54) | / | 0 | / | 0 | 42 |
| Example 18 | 2 | (56) | / | 0 | / | 0 | 28 |
| Example 19 | 4 | (32) | / | 0 | / | 0 | 88 |
| Example 20 | 8 | (32) | / | 0 | / | 0 | 84 |
| Example 21 | 12 | (32) | / | 0 | / | 0 | 55 |
| Example 22 | 1 | (32) | / | 0 | / | 0 | 22 |
| Example 23 | 15 | (32) | / | 0 | / | 0 | 30 |
| Example 24 | 8 | (32) | TTE | 0.5:1 | / | 0 | 102 |
| Example 25 | 8 | (32) | TTE | 2:1 | / | 0 | 154 |
| Example 26 | 8 | (32) | TTE | 3:1 | / | 0 | 174 |
| Example 27 | 8 | (32) | TTE | 5:1 | / | 0 | 135 |
| Example 28 | 8 | (32) | BZ | 3:1 | / | 0 | 168 |
| Example 29 | 8 | (32) | TTE | 3:1 | VC | 0.05: 1 | 192 |
| Example 30 | 8 | (32) | TTE | 3:1 | VC | 0.1: 1 | 235 |
| Example 31 | 8 | (32) | TTE | 3:1 | VC | 0.5: 1 | 150 |
| Example 32 | 8 | (32) | TTE | 3:1 | VEC | 0.1: 1 | 221 |
| Comparative Example 1 | 2 | Acetonitrile | None | 0 | / | 0 | 0 |
| Comparative Example 2 | 8 | Acetonitrile | None | 0 | / | 0 | 15 |
| Comparative Example 3 | 2 | Adiponitrile | / | 0 | / | 0 | 0 |

| | | | | | | | |
|---|---|---|---|---|---|---|---|
| Note: in Table 1, "/" indicates the absence of the substance or the parameter, and 0 cycles indicates that the performance is too poor to successfully obtain accurate test data; TTE: 1,1,2,2-tetrafluoroethyl-2,2,2-trifluoroethyl; BZ: benzene; VC: vinylene carbonate; VEC: vinyl ethylene carbonate. | | | | | | | |

Analysis of data in Table 1: as can be seen from the results of comparing Examples 1-32 with Comparative Examples 1-3, the cycle performance of the secondary battery can be improved by using the nitrile solvent with a specific structure described in the present application.

The technical features of the examples described above may be combined in any manner. For brevity, not all possible combinations of the technical features in the above examples are described. However, as long as no contradiction exists in the combinations of the technical features, such combinations should be considered to be within the scope of the specification.

The examples described above only represent several embodiments of the present disclosure. Although the descriptions of these examples are relatively specific and detailed, they should not be construed as limiting the patent scope of the present disclosure. It should be noted that various changes and modifications can be made by those skilled in the art without departing from the concept of the present disclosure, and these changes and modifications all shall fall within the protection scope of the present disclosure. Therefore, the protection scope of this patent shall be subject to the appended claims, and the specification and drawings can be used to explain the content of the claims.

## Claims

1. An electrolytic solution, wherein components of the electrolytic solution comprise a nitrile solvent, and the nitrile solvent comprises a compound represented by formula (1): 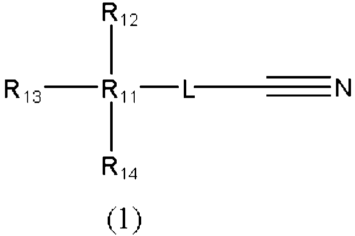
wherein, L is selected from any one of C(R₁R₂), O, Si(R₃R₄), B(R₅), P(R₆R₇R₈), and N(R₉); R₁-R₉ are each independently selected from any one of H, halogen, substituted or unsubstituted alkyl with 1-3 carbon atoms, alkoxy with 1-3 carbon atoms, amine group, and cyano, and R₁ and R₂ are not cyano and H;
R₁₁ is an alkane chain containing 1-4 carbon atoms;
R₁₂-R₁₄ are each independently selected from any one of H, halogen, substituted or unsubstituted alkyl with 1-3 carbon atoms, alkoxy with 1-3 carbon atoms, amine group, and cyano; when L is selected from C(R₁R₂), R₁₂-R₁₄ are not cyano.

2. The electrolytic solution according to claim 1, wherein the nitrile solvent comprises a compound represented by formula (1-1):

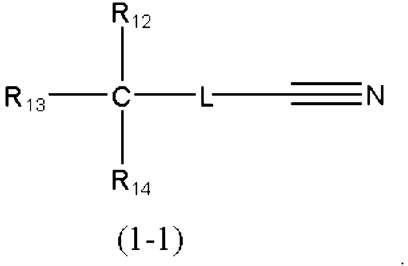

3. The electrolytic solution according to any one of claims 1-2, wherein L is selected from any one of C(R₁R₂), Si(R₃R₄), and N(R₉).

4. The electrolytic solution according to claim 1, wherein the compound represented by formula (1) satisfies at least one of the following conditions (1)-(3):
(1) R₁-R₉ are each independently selected from any one of halogen, alkyl with 1-3 carbon atoms, halogen-substituted alkyl with 1-3 carbon atoms, alkoxy with 1-3 carbon atoms, and cyano;
(2) R₁ and R₂ are not H at the same time, R₃ and R₄ are not H at the same time, R₆, R₇, and R₈ are not H at the same time, and R₅ and R₉ are not H; and
(3) R₁₂-R₁₄ are each independently selected from any one of H, halogen, alkyl with 1-3 carbon atoms, halogen-substituted alkyl with 1-3 carbon atoms, alkoxy with 1-3 carbon atoms, and cyano.

5. The electrolytic solution according to any one of claims 1-4, wherein the nitrile solvent comprises at least one of compounds represented by formula (1a) to formula (1f): 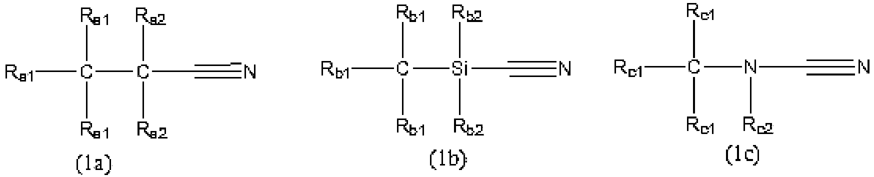 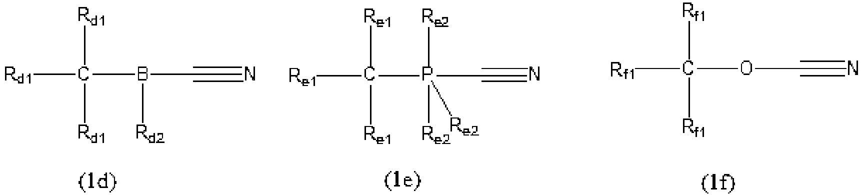
wherein, each Rₐ₁, each R_{b1}, each R_{c1}, each R_{d1}, each Rₑ₁, and each R_{f1} are each independently selected from any one of H, halogen, alkyl with 1-3 carbon atoms, halogen-substituted alkyl with 1-3 carbon atoms, alkoxy with 1-3 carbon atoms, and cyano, and each Rₐ₁ is not cyano;
each Rₐ₂, each R_{b2}, each R_{c2}, each R_{d2}, and each Rₑ₂ are each independently selected from any one of halogen, alkyl with 1-3 carbon atoms, halogen-substituted alkyl with 1-3 carbon atoms, and alkoxy with 1-3 carbon atoms.

6. The electrolytic solution according to claim 5, wherein the nitrile solvent satisfies at least one of the following conditions (1)-(6):
(1) at least one Rₐ₂ is alkoxy with 1-3 carbon atoms;
(2) each R_{b2} is independently selected from any one of alkyl with 1-3 carbon atoms and alkoxy with 1-3 carbon atoms;
(3) each R_{c1} is independently selected from alkyl with 1-3 carbon atoms;
(4) each R_{d1} is independently selected from alkyl with 1-3 carbon atoms;
(5) each Rₑ₁ is independently selected from alkoxy with 1-3 carbon atoms; and
(6) each R_{f1} is independently selected from any one of H and alkyl with 1-2 carbon atoms.

7. The electrolytic solution according to any one of claims 1-6, wherein the nitrile solvent comprises at least one of compounds represented by formula (11) to formula (58):

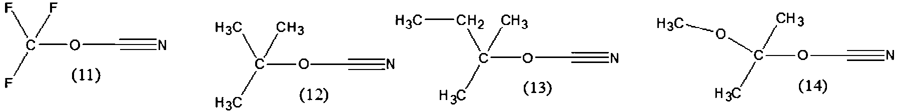

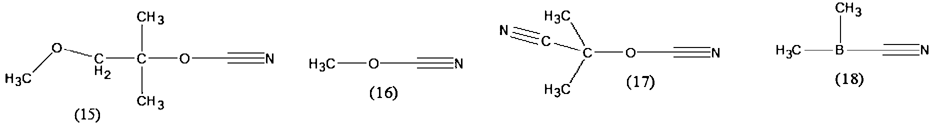

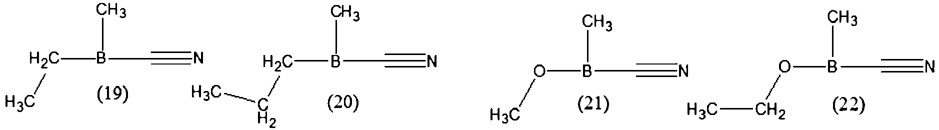

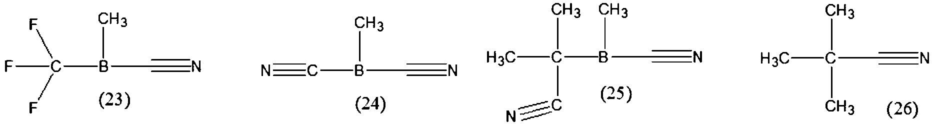

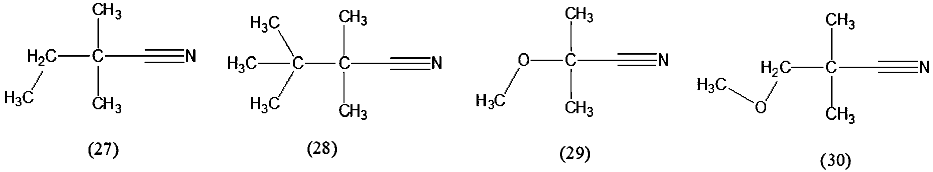

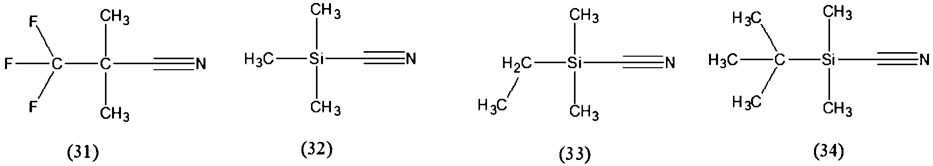

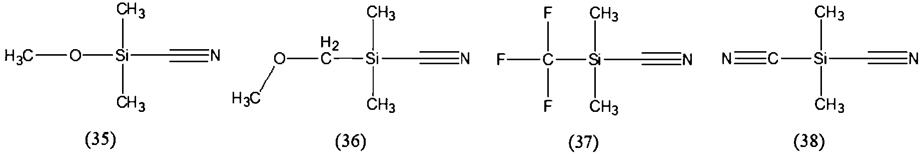

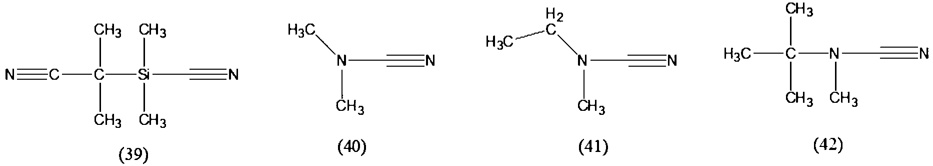

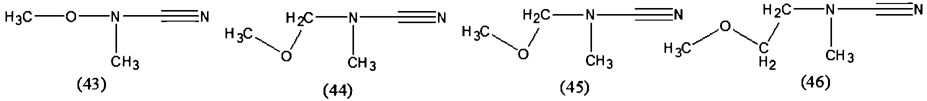

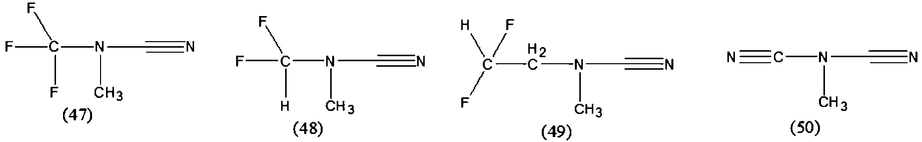

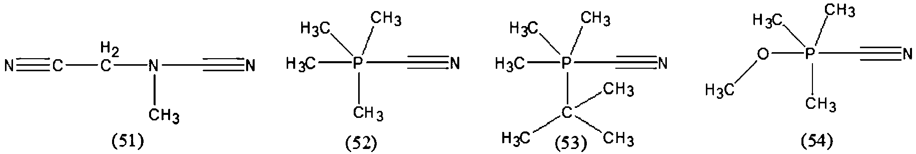

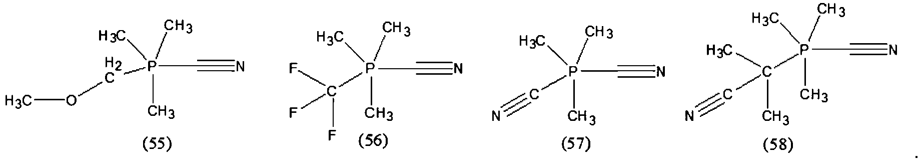

8. The electrolytic solution according to any one of claims 1-7, wherein the components of the electrolytic solution further comprise an electrolyte salt, and a molar volume ratio of the electrolyte salt to the nitrile solvent is 1 mol/L to 15 mol/L.

9. The electrolytic solution according to claim 8, wherein the electrolyte salt satisfies at least one of the following conditions (1)-(2):
(1) in the electrolytic solution, the molar volume ratio of the electrolyte salt to the nitrile solvent is 2 mol/L to 12 mol/L; and
(2) the electrolyte salt comprises a lithium-ion electrolyte salt.

10. The electrolytic solution according to any one of claims 1-9, wherein the components of the electrolytic solution further comprise a film-forming additive, and the film-forming additive comprises an unsaturated cyclic carbonate-based film-forming additive.

11. The electrolytic solution according to claim 10, wherein a molar ratio of the film-forming additive to the nitrile solvent is (0.05-0.5):1.

12. The electrolytic solution according to any one of claims 10-11, wherein the film-forming additive comprises at least one of vinylene carbonate, vinyl ethylene carbonate, fluoroethylene carbonate, ethylene sulfate, propylene sulfate, and ethylene sulfite.

13. A secondary battery, wherein the secondary battery comprises the electrolytic solution according to any one of claims 1-12.

14. The secondary battery according to claim 13, wherein the secondary battery is a lithium metal secondary battery.

15. An electric device, wherein the electric device comprises the secondary battery according to any one of claims 13-14.
